# EUROPEAN PATENT APPLICATION

(11) **EP 4 368 216 A1**
(43) Date of publication of application: **15.05.2024**
(21) Application number: 23709305.9
(22) Date of filing: 06.01.2023
(51) Int. Cl.: A61L 27/58, A61L 27/44, A61L 31/06, A61L 31/14, A61B 17/56

(54) **COMPOSITION FOR DEGRADABLE JOINT BALLOON AND USE THEREOF, AND DEGRADABLE JOINT BALLOON AND PREPARATION METHOD THEREFOR**

(30) Priority: 15.09.2022 CN 202211123650
(71) Applicant: STAR SPORTS MEDICINE CO., LTD., Beijing 100176 (CN)
(72) Inventor: DONG, Wenxing, Beijing 100176 (CN); QU, Yuanyuan, Beijing 100176 (CN)
(74) Representative: Vesterinen, Jussi Tapio
(86) International application number: PCT/CN2023/070854
(87) International publication number: WO 2024/055490

(57) **Abstract**

The present application relates to the technical field of medical apparatus and instruments, and particularly discloses a composition for degrading a joint balloon and use thereof as well as the degradable joint balloon and a preparation method thereof. The composition for degrading the joint balloon comprises the following components in parts by weight: 90-99.9 parts of degradable polymer material, 0.1-10 parts of nano hydroxide and 0.1-2 parts of dispersant; the nano hydroxide is selected from nano magnesium hydroxide and nano calcium hydroxide. Provided is use of the composition for degrading the joint balloon in preparation of the degradable joint balloon. The degradable joint balloon is prepared by using the composition. The preparation method of the degradable joint balloon is selected from a dip coating method, an electrostatic spinning method, an extrusion method, an injection molding method, a blow molding method and a mould pressing method. The present application utilizes nano magnesium hydroxide, thereby reducing acid inflammation induced by the joint balloon.

## Description

### TECHNICAL FIELD

The present application relates to the technical field of medical apparatus and instruments, particularly to a composition for degrading a joint balloon and use thereof as well as the degradable joint balloon and a preparation method thereof.

### BACKGROUND

A joint balloon is used for treating joint injury diseases as a filler, and its mechanism of action is that the joint balloon is implanted into a joint cavity (gap) to take supporting and lubricating effects, specifically, the joint balloon is filled with a gas, a liquid or a gel so that the joint balloon has enough elasiticity to ensure the smooth and friction-free motion between bones, and the damage of the joint is restored by replacing damaged joint balloon or transient supporting. For example, a rotator cuff balloon, which fills the joint balloon between the shoulder peak and the humerus, can isolate the tom shoulder sleeve and prevent the shoulder sleeve wound from colliding with the shoulder peak, thus alleviating the pain. After reconstructing the shoulder-brachial distance, the arm strength of joint lifting can be increased, and the shoulder joint mobility is improved.

The materials for preparing the joint balloon mainly include a degradable polymer material and a non-degradable polymer material. Among them, the degradable polymer material, such as (α-hydroxyl ester), increasingly draws people's attentions because it is completely degraded and absorbed after the joint injury is recovered. However, the degradable polymer material produces acidic byproducts in the process of degradation, and the acidic byproducts easily induce tissue inflammation, so that the application of the joint balloon is limited to a certain extent.

### SUMMARY

To reduce the acidic inflammation induced by the joint balloon, the present application provides a composition for degrading a joint balloon and use thereof as well as the degradable joint balloon and a preparation method thereof.

In the first aspect, the composition for degrading the joint balloon provided by the present application adopts the following technical solution:

Provided is a composition for degrading a joint balloon, comprising the following components in parts by weight:

90-99.9 parts of degradable polymer material, 0.1-10 parts of nano hydroxide and 0.1-2 parts of dispersant;

the nano hydroxide being selected from nano magnesium hydroxide and nano calcium hydroxide.

In some embodiments, the use amount of the degradable polymer material is 91 weight parts, 92 weight parts, 93 weight parts, 94 weight parts, 95 weight parts, 96 weight parts, 97 weight parts, 98 weight parts, 98.1 weight parts, 98.4 weight parts, 98.6 weight parts, 98.8 weight parts or 99 weight parts.

In some embodiments, the use amount of the nano hydroxide is 0.2 weight part, 0.3 weight part, 0.4 weight part, 0.5 weight part, 0.6 weight part, 0.7 weight part, 0.8 weight part, 0.9 weight part, 1.0 weight part, 1.1 weight parts, 1.2 weight parts, 1.3 weight parts, 1.4 weight parts, 1.5 weight parts, 1.6 weight parts, 1.7 weight parts, 1.8 weight parts, 1.9 weight parts, 2.0 weight parts, 3.0 weight parts, 4.0 weight parts, 5.0 weight parts, 6.0 weight parts, 7.0 weight parts, 8.0 weight parts or 9.0 weight parts.

In some embodiments, the use amount of the dispersant is 0.2 weight part, 0.3 weight part, 0.4 weight part, 0.5 weight part, 0.6 weight part, 0.7 weight part, 0.8 weight part, 0.9 weight part, 1.0 weight part, 1.1 weight parts, 1.2 weight parts, 1.3 weight parts, 1.4 weight parts, 1.5 weight parts, 1.6 weight parts, 1.7 weight parts, 1.8 weight parts or 1.9 weight parts.

In some embodiments, the nano hydroxide has a particle size of 10-200 nm, such as 10 nm, 20 nm, 30 nm, 40 nm, 50 nm, 60 nm, 70 nm, 80 nm, 90 nm, 100 nm, 110 nm, 120 nm, 130 nm, 140 nm, 150 nm, 160 nm, 170 nm, 180 nm, 190 nm and 200 nm.

In some embodiments, the nano hydroxide comprises a nano hydroxide with a particle size of 20 nm, a nano hydroxide with a particle size of 50 nm and a nano hydroxide with a particle size of 100 nm in a weight ratio of (2-4): (4-6): (1-3). For example, the nano hydroxide comprises a nano hydroxide with a particle size of 20 nm, a nano hydroxide with a particle size of 50 nm and a nano hydroxide with a particle size of 100 nm in a weight ratio of 3:5:2.

In some embodiments, the degradable polymer material has an intrinsic viscosity of η=1.3-1.7 dl/g, such as 1.35 dl/g, 1.4 dl/g, 1.45 dl/g, 1.5 dl/g, 1.55 dl/g, 1.6 dl/g and 1.65 dl/g.

In some embodiments, the degradable polymer material is selected from copolymers or blends of polyglycolic acid, polylactic acid, polylactic acid-glycolic acid, polycaprolactone, polyethylene terephthalate and poly(L-lactide-ε-caprolactone).

In some embodiments, the degradable polymer material is poly(L-lactide-ε-caprolactone). Specifically, the poly(L-lactide-ε-caprolactone) is formed by polymerizing L-lactide with ε-caprolactone in a molar ratio of (60-80):(20-40), such as 60:40, 65:35, 70:30, 75:25 and 80:20.

In some embodiments, the dispersant is selected from an anionic dispersant, a cationic dispersant and a polymer dispersant. The anionic dispersant can be stearate, such as calcium stearate. The cationic dispersant can be an amine salt. The polymer dispersant can be carboxymethyl cellulose (CMC) and hydroxyethyl cellulose (HEC).

In the second aspect, the present application provides use of the composition for a degradable joint balloon in preparation of the degradable joint balloon.

In the third aspect, the present application provides a degradable joint balloon. The adopted technical solution is as follows:

Provided is a degradable joint balloon, the degradable joint balloon being prepared by utilizing the composition.

In some embodiments, the degradable joint balloon has a thickness of 50-200 µm, such as 60 µm, 70 µm, 80 µm, 90 µm, 100 µm, 110 µm, 120 µm, 130 µm, 140 µm, 150 µm, 160 µm, 170 µm, 180 µm and 190 µm.

In the fourth aspect, the present application provides a preparation method of a degradable joint balloon. The adopted technical solution is as follows:

Provided is a preparation method of a degradable joint balloon, the preparation method being selected from a dip coating method, an electrostatic spinning method, an extrusion method, an injection molding method, a blow molding method and a mould pressing method.

In some embodiments, when the preparation method is the dip coating method, the preparation method comprises the following steps:
(1) preparing the composition into a dispersion by using an organic solvent;
(2) immersing a balloon molding model into the dispersion, subsequently taking out the balloon molding model, and then allowing the composition to form a film on the surface of the balloon forming model after the organic solvent in the dispersion adhered to the surface of the balloon molding model is evaporated;
   performing step (2) at least once; and
(3) demoulding and drying to obtain the degradable joint balloon.

In some embodiments, in step (1), the specific preparation process of the dispersant is as follows: the degradable polymer material is dissolved into the organic solvent, and the nano hydroxide and the dispersant are then added and evenly dispersed to obtain the dispersion.

In some embodiments, in step (1), a weight ratio of the degradable polymer material to the organic solvent in the composition is (1-20): (80-99), further (1-20): (80-90), such as 10:90, 15:85 and 20:80.

In some embodiments, the organic solvent is selected from 2,2,2-trifluoroethanol, hexafluoroisopropanol, dichloromethane and chloroform.

In some embodiments, a material for fabricating the balloon forming model is selected from gelatin and low-temperature agar.

In some embodiments, in step (2), the specific fabrication process of the balloon forming model is as follows: the material for fabricating the balloon forming model is dissolved into hot water of 60-70°C (for example, 61°C, 62°C, 63°C, 64°C, 65°C, 66°C, 67°C, 68°C and 69°C) to obtain 3-10 wt% material solution (for example, 4wt%, 5wt%, 6wt%, 7wt%, 8wt% and 9wt%), and the material solution is poured into a mould when it is hot and then cooled and sized, and then demoulded to obtain the balloon forming model.

In some embodiments, in step (3), the specific operation process of the demoulding is as follows: the balloon forming model with the degradable joint balloon formed on the surface is melted into a liquid through thermal treatment, and the melted balloon forming model is extruded from the degradable joint balloon. Where, the process of the thermal treatment is as follows: the balloon forming model with the degradable joint balloon formed on the surface is immersed into hot water. Where, the temperature of the hot water is 60-70°C, such as 61°C, 62°C, 63°C, 64°C, 65°C, 66°C, 67°C, 68°C and 69°C.

In some embodiments, in step (3), the drying is vacuum drying.

In some embodiments, the content of the residual organic solvent in the degradable joint balloon is < 0.3 ppm.

In summary, the present application has the following beneficial effects:

First, the acidic byproducts produced during the degradation of the degradable polymer material cause the reduction in the pH value of the buffer solution, the solubility of nano magnesium hydroxide in water is very small, but the part of nano magnesium hydroxide that is dissolved into water can be fully ionized, and the OH⁻ produced by the ionization of nano magnesium hydroxide can neutralize the H⁺ in the buffer solution; therefore, nano magnesium hydroxide can continuously ionize OH⁻, and can continuously adjust the H⁺ in the neutralization buffer solution to adjust the reduction in pH value caused by the degradable polymer material during the degradation, thereby reducing the acid inflammation induced by the degradable joint balloon.

Second, in the present application, it is preferred to use the combination of nanometer magnesium hydroxide with a particle size of 20 nm, nanometer magnesium hydroxide with a particle size of 50 nm and nanometer magnesium hydroxide with a particle size of 100 nm, thereby optimizing the balloon bursting pressure and flexibility of the degradable joint balloon.

### BRIEF DESCRIPTION OF THE DRAWINGS

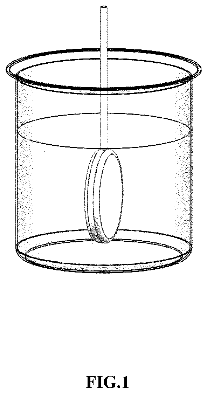

FIG. 1 is a schematic diagram of a dip coating method according to the present application.

### DETAILED DESCRIPTION OF THE EMBODIMENTS

Next, the present application will be further described in detail in combination with embodiments.

### Fabrication of balloon forming model

The present application takes gelatin as an example to discuss the fabrication process of the balloon forming model.

The specific fabrication process of the balloon forming model is as follows: gelatin is dissolved into 65°C hot water to obtain a 5wt% gelatin solution, and the gelatin solution is poured into a mould when it is hot and the gelatin solution is cooled and sized, and then demoulded to obtain the balloon forming model.

Where, the present application takes a balloon forming model with an oval shape of 60 mm × 70 mm and a thickness of 6.5 mm as an example to conduct the following experiment.

### Preparation of degradable joint balloon

The specific preparation method of the degradable joint balloon comprises the following steps:
(1) a degradable polymer material is dissolved into an organic solvent, a nano hydroxide and a dispersant are then added and the above materials are subjected to ultrasonic dispersion for 30 min (stop for 10 min every ultrasonic dispersion for 10 min, for 50 min in total) to obtain the dispersion;
(2) the dispersion is dip coated onto the surface of the above obtained balloon forming model using a dip coating method to form a film;
   as shown in FIG. 1, the specific operation process of the dip coating method is as follows: the balloon forming model is immersed into the dispersion, and the balloon forming model is then taken out, the composition formed a film on the surface of the balloon forming model after the organic solvent in the dispersion that is adhered to the surface of the balloon forming model is evaporated;
   step (2) is performed at least once;
(3) the degradable joint balloon is obtained by demoulding and vacuum drying;
   where, the specific operation process of the demoulding is as follows: the balloon forming model with the degradable joint balloon on the surface is immersed into 65°C hot water to melt the balloon forming model into a liquid, and the melted balloon forming model is extruded from the degradable joint balloon.

The shape and size of the degradable joint balloon of the present application depend on the balloon forming model. The present application is discussed by taking the degradable joint balloon with a thickness of 100 µm as an example. To make the degradable joint balloon reach the desired thickness, the thickness of the degradable joint balloon is controlled as 100 µm by selecting specific dip coating times in step (2).

### Performance detection

Performance detection of dispersion: the scraper fineness of the dispersion is detected by using a scraper fineness meter.

Performance detection of degradable joint balloon:
(1) compressive strength test of balloon: the balloon is injected with 25 mL of normal saline and then sealed, pressure test is performed at the speed of 1 mm/min until the balloon is burst, and the bursting pressure of the balloon is recorded.
(2) bending performance test of balloon: the balloon is rolled along the long axis of the balloon, the rolled balloon is cylindrical, and the rolled balloon is put into a cylindrical sleeve with an inner diameter of 6 mm (the axis of the rolled balloon is parallel to the axis of the cylindrical sleeve). The convolution and loading of the balloon are observed to evaluate the bending performance of the balloon as "good" or "poor".
   The criterion for evaluation as "excellent" is that the rolled balloon can be completely put into the cylindrical sleeve, and the balloon has no crease or crack.
   The criterion for evaluation as "poor" is that the rolled balloon cannot be put into the cylindrical sleeve (that is, the diameter of the rolled balloon exceeds the inner diameter of the cylindrical sleeve), or the balloon has creases and cracks.
(3) In-vitro accelerated degradation performance test of balloon: the balloon is immersed into PBS buffer solution (pH value (before)=7.40), then the PBS buffer solution immersed with the balloon is placed in an oven at 50°C for accelerated degradation (lasting for 12 weeks), the pH value (after) of the PBS buffer solution after 12-week in vitro accelerated degradation of the balloon is measured, and the reduction ΔpH in the pH values before and after 12-week in vitro accelerated degradation of the balloon is calculated.

Calculation formula: ΔpH=pH value (before)-pH value (after).

The degradable polymer material used in examples of the present application and comparative examples is poly(L-lactide-ε-caprolactone); where, the poly(L-lactide-ε-caprolactone) is formed by polymerizing L-lactide with ε-caprolactone in a molar ratio of 70:30, and the intrinsic viscosity of poly(L-lactide-ε-caprolactone) is 1.5 dl/g.

### Examples 1-7 and comparative example 1

Examples 1-7 differ from comparative example 1 in that the use amounts of the degradable polymer material, the nano hydroxide and the dispersant are different.

**Table 1 Ingredient list and performance detection results in examples 1-7 and comparative example 1**

| Ingredient and performance detection | Examples | | | | | | | Comparative example |
|---|---|---|---|---|---|---|---|---|
| | 1 | 2 | 3 | 4 | 5 | 6 | 7 | 1 |
| Poly(L-lactide-ε-caprolactone) | 99.1 | 98.8 | 98.6 | 98.4 | 98.4 | 98.1 | 97.9 | 100 |
| Nano hydroxide with a particle size of 50 nm | 0.5 | 1 | 1 | 1 | 1.2 | 1.5 | 1.5 | - |
| Calcium stearate | 0.4 | 0.2 | 0.4 | 0.6 | 0.4 | 0.4 | 0.6 | - |
| Weight ratio of poly(L-lactide-ε-caprolactone) to dichloromethane | 15:85 | 15:85 | 15:85 | 15:85 | 15:85 | 15:85 | 15:85 | 15:85 |
| In step (2), times of dip coating | 2 | 2 | 2 | 2 | 2 | 2 | 2 | 3 |
| Thickness of balloon, µm | 100 | 100 | 100 | 100 | 100 | 100 | 100 | 100 |
| Scraper fitness of dispersion, µm | 5 | 7.5 | 5 | 5 | 10 | 10 | 10 | 0 |
| Bursting pressure of balloon, N | 728 | 776 | 829 | 808 | 849 | 878 | 897 | 694 |
| Convolution performance of balloon | Good | Good | Good | Good | Poor | Poor | poor | Good |
| pH value (after) | 6.80 | 6.87 | 6.92 | 6.90 | 6.96 | 7.04 | 7.05 | 6.72 |
| ΔpH | 0.60 | 0.53 | 0.48 | 0.50 | 0.44 | 0.36 | 0.35 | 0.68 |
| Note: "-" represents that the use amount is zero | | | | | | | | |

It can be seen from Table 1 that the degradable joint balloon in the present application not only increases the bursting pressure of the balloon, but also decreases the reduction ΔpH in the pH value after 12-week in vitro accelerated degradation of the balloon. This is because the acidic byproducts produced during the degradation of the degradable polymer material cause the reduction in the pH value of the buffer solution, the solubility of nano magnesium hydroxide in water is very small, but the part of nano magnesium hydroxide that is dissolved into water can be fully ionized, and the OH⁻ produced by the ionization of nano magnesium hydroxide can neutralize the H⁺ in the buffer solution; therefore, nano magnesium hydroxide can continuously ionize OH⁻, and can continuously adjust the H⁺ in the neutralization buffer solution to adjust the reduction in pH value caused by the degradable polymer material during the degradation, thereby reducing the acid inflammation induced by the degradable joint balloon.

By comparing examples 1 and 3 and examples 5-6, it can be seen that with the increase of the use amount of nano hydroxide, the reduction ΔpH in the pH value of the balloon after 12-week in vitro accelerated degradation becomes smaller and smaller, and the bursting pressure of the balloon becomes larger and larger. However, with the increase of the use amount of nano hydroxide, the flexibility of the degradable joint balloon becomes worse, and the convolution performance of the balloon changes from "good" to "poor".

By comparing examples 2-4, it can be seen that the dispersant can be used as a bridge between nano hydroxide and the degradable polymer material to make the nano hydroxide disperse more evenly, so as to improve the balloon bursting pressure of the degradable joint balloon. However, the excessive amount of the dispersant can lead to the increase in small and medium-sized molecular materials in the dispersion, and the bursting pressure of the balloon is slightly reduced.

### Examples 8-14

Compared with example 3, the difference is that the particle size of nano magnesium hydroxide used in examples 8-14 is different.

**Table 2 Particle sizes and performance detection results of nano magnesium hydroxide used in examples 8-14**

| Particle sizes of nano magnesium hydroxide (unit: g) | Example | | | | | | |
|---|---|---|---|---|---|---|---|
| | 8 | 9 | 10 | 11 | 12 | 13 | 14 |
| Nano magnesium hydroxide with a particle size of 20 nm | 1 | - | - | 0.3 | - | 0.6 | 0.375 |
| Nano magnesium hydroxide with a particle size of 50 nm | - | - | - | 0.5 | 0.714 | - | 0.625 |
| Nano magnesium hydroxide with a particle size of 100 nm | - | 1 | - | 0.2 | 0.286 | 0.4 | - |
| Nano magnesium hydroxide with a particle size of 200 nm | - | - | 1 | - | - | - | - |
| In step (2), times of dip coating | 2 | 2 | 2 | 2 | 2 | 2 | 2 |
| Thickness of balloon, µm | 100 | 100 | 100 | 100 | 100 | 100 | 100 |
| Scraper fitness of dispersion, µm | 5 | 12.5 | 15 | 12.5 | 12.5 | 12.5 | 5 |
| Bursting pressure of balloon, N | 836 | 754 | 735 | 867 | 826 | 839 | 849 |
| Convolution performance of balloon | Poor | Good | Good | Good | Good | Poor | Poor |
| pH value (after) | 6.93 | 6.92 | 6.92 | 6.92 | 6.92 | 6.92 | 6.92 |
| ΔpH | 0.47 | 0.48 | 0.48 | 0.48 | 0.48 | 0.48 | 0.48 |

It can be seen from Table 2 that the use of nano hydroxides with different particle sizes mainly affects the bursting pressure of the balloon and the balloon convolution performance of the degradable joint balloon, but has little effect on the reduction ΔpH in pH value after 12-week in vitro accelerated degradation of the balloon.

By comparing examples 3, 8 and 10, it can be seen that with the increase of the particle size of the used nano hydroxide, the bursting pressure of the degradable joint balloon becomes smaller and smaller, but the flexibility of the degradable joint balloon becomes better.

By comparing examples 3, 8, 9 and 11, it can be seen that the combination of a nano magnesium hydroxide with a particle size of 20 nm, a nano magnesium hydroxide with a particle size of 50 nm and a nano magnesium hydroxide with a particle size of 100 nm can optimize the balloon bursting pressure of the degradable joint balloon.

### Examples 15-16

Compared with example 3, the difference is the weight ratio of poly(L-lactide-ε-caprolactone) to dichloromethane in examples 15-16.

**Table 3 Weight ratios of poly(L-lactide-ε-caprolactone) to dichloromethane and performance detection results in examples 15-16**

| Example | 15 | 16 |
|---|---|---|
| Weight ratio of poly(L-lactide-ε-caprolactone) to dichloromethane | 10:90 | 20:80 |
| In step (2), times of dip coating | 4 | 2 |
| Thickness of balloon, µm | 100 µm | 100 µm |
| Scraper fitness of dispersion, µm | 5 | 10 |
| Bursting pressure of balloon, N | 821 | 779 |
| Convolution performance of balloon | Good | Good |
| pH value (after) | 6.92 | 6.92 |
| ΔpH | 0.48 | 0.48 |

It can be seen from Table 3 that the different concentrations of poly(L-lactide-ε-caprolactone) in the dispersion cause the slightly different film-forming properties of the degradable joint balloon, a proper concentration is conducive to homogeneous texture of poly(L-lactide-ε-caprolactone) during the film formation while the degradable joint balloon has good mechanical property.

It is understood that the above embodiments are only exemplary embodiments used for illustrating the principle of the present disclosure, but not limiting the present disclosure. For persons of ordinary skill in the art, various deformations and improvements can be made without departing from the spirit and essences of the present disclosure, and these deformations and improvements are deemed to be within the protective scope of the present disclosure.

## Claims

1. A composition for degrading a joint balloon, the composition comprising the following components in parts by weight:
90-99.9 parts of degradable polymer material, 0.1-10 parts of nano hydroxide and 0.1-2 parts of dispersant;
the nano hydroxide being selected from nano magnesium hydroxide and nano calcium hydroxide.

2. The composition according to claim 1, wherein the nano hydroxide has a particle size of 10-200 nm.

3. The composition according to claim 1, wherein the nano hydroxide comprises a nano hydroxide with a particle size of 20 nm, a nano hydroxide with a particle size of 50 nm and a nano hydroxide with a particle size of 100 nm in a weight ratio of (2-4): (4-6): (1-3).

4. The composition according to claim 1, wherein the degradable polymer material has an intrinsic viscosity of η=1.3-1.7 dl/g.

5. The composition according to claim 1, wherein the degradable polymer material is selected from copolymers or blends of polyglycolic acid, polylactic acid, polylactic acid-glycolic acid, polycaprolactone, polyethylene terephthalate and poly(L-lactide-ε-caprolactone).

6. Use of the composition according to any one of claims 1-5 in preparation of the degradable joint balloon.

7. A degradable joint balloon, the degradable joint balloon being prepared by utilizing the composition according to any one of claims 1-5.

8. A preparation method of a degradable joint balloon according to claim 7, wherein the preparation method is selected from a dip coating method, an electrostatic spinning method, an extrusion method, an injection molding method, a blow molding method and a mould pressing method.

9. The preparation method according to claim 8, wherein when the preparation method is the dip coating method, the preparation method comprises the following steps:
(1) preparing the composition into a dispersion by using an organic solvent;
(2) immersing a balloon molding model into the dispersion, subsequently taking out the balloon molding model, and then allowing the composition to form a film on the surface of the balloon forming model after the organic solvent in the dispersion adhered to the surface of the balloon molding model is evaporated;
performing step (2) at least once; and
(3) demoulding and drying to obtain the degradable joint balloon.

10. The preparation method according to claim 9, wherein in step (1), a weight ratio of the degradable polymer material to the organic solvent in the composition is (1-20): (80-99).
